# FASCICULE DE BREVET EUROPEEN

(11) **EP 4 539 726 B1**
(45) Date de publication et mention de la délivrance du brevet: **24.12.2025**
(21) Numéro de dépôt: 23732080.9
(22) Date de dépôt: 09.06.2023
(51) Int. Cl.: A61B 3/11

(54) **LENTILLE SCLERALE INSTRUMENTEE ET DISPOSITIF ASSOCIE, EMBARQUE DANS LA LENTILLE OU NON, POUR LA MESURE DU DIAMETRE DE PUPILLE D'UN OEIL**
INSTRUMENTIERTE SKLERALINSE UND ZUGEHÖRIGE, OPTIONAL IN DER LINSE ANGEORDNETE VORRICHTUNG ZUR MESSUNG DES PUPILLENDURCHMESSERS EINES AUGES
INSTRUMENTED SCLERAL LENS AND ASSOCIATED DEVICE, OPTIONALLY FITTED IN THE LENS, FOR MEASURING THE PUPIL DIAMETER OF AN EYE

(30) Priorité: 14.06.2022 FR 2205749
(43) Date de publication de la demande: 23.04.2025
(73) Titulaire: Institut Mines Telecom, 91120 Palaiseau (FR)
(72) Inventeur: DE BOUGRENET DE LA TOCNAYE, Jean-Louis, 29820 GUILERS (FR); NOURRIT, Vincent, 29200 BREST (FR); HEGGARTY, Kevin, 29290 LANRIVOARE (FR)
(74) Mandataire: Ipsilon
(86) Numéro de dépôt international: PCT/EP2023/065536
(87) Numéro de publication internationale: WO 2023/242067

(56) Documents cités:
- WO-A1-2017/025325
- WO-A1-90/12534

## Description

### Domaine technique

La présente invention concerne une lentille de contact instrumentée, et plus particulièrement une lentille sclérale.

La lentille selon l'invention peut être un système complètement autonome et embarqué sur au moins un œil d'un individu.

L'invention vise en particulier à mesurer de manière automatique le diamètre de pupille d'un individu grâce à une lentille sclérale qui embarque tout ou partie des composants électroniques formant un pupillomètre.

Les applications de la lentille sclérale selon l'invention sont nombreuses parmi lesquelles on peut citer celles de la médecine telles que l'addictologie, l'anesthésie, l'ophtalmologie, la neurologie, la psychologie, la pharmacologie, la réanimation, ou la toxicologie. De manière générale, l'invention qui permet de mesurer la taille d'une pupille trouve application dans tous les domaines de mesure des processus cognitifs du cerveau humain.

### Technique antérieure

WO 2017/025325 Al divulgue un dispositif de pupillométrie avec lentille de contact comprenant un support transparent 12, des moyens d'illumination 30, des photodétecteurs 20 pour capturer lumière réfléchie, un processeur 42 et une antenne 48 disposés sur le support 12 (page 36, lignes 19-21).

WO 90/12534 Al divulgue une lentille sclérale 1 avec un système optique externe monté sur support 6; des fibres optiques 21/22 attachées au support 6 (page 17) pour illumination/réception, un détecteur CCD externe (page 18), une architecture modulaire avec support saillant empêchant la fermeture de l'œil.

Il est connu que la pupille d'un sujet humain est un indicateur de l'état physiologique ou psychologique de celui-ci.

Il a ainsi été constaté en particulier que la taille d'une pupille d'un individu varie en fonction de nombreux paramètres de l'environnement et des stimulations extérieures, tels que la luminosité ambiante, l'absorption de drogues ou d'antalgiques, ainsi que de l'état d'éveil ou l'état émotionnel de l'individu ou encore sa sensibilité à la douleur.

Les mesures de la pupille peuvent être réalisées à l'aide d'un pupillomètre configuré pour mesurer les variations de taille ou de forme de la pupille.

Un pupillomètre est un dispositif permettant de mesurer la taille de l'ouverture de la pupille à divers stades (au repos, en myosis, en mydriase) et ce, sans mettre d'objet en contact avec le globe oculaire.

Les pupillomètres connus intègrent le plus souvent un éclairage, et une caméra. Ils permettent de recueillir, pour chaque œil, des images traitées en temps réel notamment le diamètre pupillaire, l'amplitude et la vitesse de contraction ou de dilatation : [1].

Un pupillométrie dynamique standard peut faire jusqu'à 30 mesures par seconde et un pupillomètre rapide jusqu'à 200.

Le traitement d'images est plus ou moins complexe selon la nature de l'information recherchée et les conditions d'éclairage ambiant.

En général, l'éclairage est réalisé par une source infrarouge qui éclaire la cornée permettant ainsi la mesure en régime scotopique, i.e. dans l'obscurité.

Les mesures en tant que telle peuvent être effectuées par différents instruments.

Le pupillomètre de Colvard utilise un système de visualisation directe avec repère gradué en millimètres.

Un pupillomètre est souvent intégré à d'autres dispositifs de mesures ophtalmiques comme des aberromètres ou topographes, par exemple ceux commercialisés sous la dénomination OPD SCAN III (Nidek) ou Wavelight ^{®} Topolyzer^{™} VARIO (Alcon). En général, le patient appuie son visage contre une partie fixe du dispositif afin que ces yeux soient isolés de la lumière ambiante qui pourrait perturber la mesure.

Certains pupillomètres, comme celle proposée dans le brevet EP2609852B1, mettent en œuvre une méthode par éclairement direct de la cornée ou de l'iris et une mesure de l'intensité réfléchie qui permet d'en déduire le diamètre pupillaire.

Cette technique présente les inconvénients majeurs de nécessiter la fermeture de l'œil et la présence d'un dispositif de communication filaire posé sur l'œil certes adapté dans le cadre d'interventions chirurgicales, mais inadapté à une mesure en condition de mobilité oculaire et de déplacement d'un individu.

Il existe donc un besoin d'améliorer encore les pupillomètres existants, notamment afin de permettre à un individu portant l'équipement nécessaire à la mesure du diamètre de sa pupille de ne pas être contraint dans sa mobilité à la fois oculaire et de déplacement, et de préférence dans un usage éventuellement ambulatoire, e.g. permettre une mesure indépendante des mouvements de l'oeil.

Le but de l'invention est de répondre au moins partiellement à ce besoin.

### Exposé de l'invention

Pour ce faire, l'invention a pour objet selon une première alternative une lentille sclérale pour la mesure du diamètre d'une pupille d'œil d'individu, comprenant:
- une membrane adaptée pour recouvrir la pupille, l'iris et au moins partiellement la sclère de l'œil ;
- une source d'illumination encapsulée dans la membrane, la source d'illumination étant adaptée pour émettre un cône ou faisceau lumineux destiné à diverger directement ou indirectement vers l'iris;
- un circuit électronique, encapsulé dans la membrane, comprenant au moins comme composants:
   au moins un photodétecteur agencé de sorte à capter le faisceau émis par la source d'illumination réfléchie par la surface de l'iris,
   un microcontrôleur, relié au photodétecteur et adapté pour convertir en données numériques les signaux électriques issus du photodétecteur et calculer le diamètre de la pupille, à partir des données numériques converties, en fonction d'une table de correspondance prédéterminée et les coder pour transmission par communication sans fil,
   une antenne pour transmettre les informations relatives au diamètre de pupille calculé par communication sans fil.

De préférence, le photodétecteur est une photodiode.

Avantageusement, l'antenne est adaptée pour transmettre les informations par communication en champs proche (NFC).

Selon un mode de réalisation avantageux, l'antenne est en outre adaptée pour réaliser une recharge électrique de la source d'illumination et/ou des composants actifs du circuit électronique.

Selon une deuxième alternative, l'invention concerne une lentille sclérale pour la mesure du diamètre d'une pupille d'oeil d'individu, comprenant:
- une membrane adaptée pour recouvrir la pupille, l'iris et au moins partiellement la sclère de l'œil ;
- une source d'illumination encapsulée dans la membrane et adaptée pour émettre un cône ou faisceau lumineux destiné à diverger directement ou indirectement vers l'iris ;
- un élément optique, encapsulé dans la membrane et agencé de sorte à capter le faisceau émis par source d'illumination réfléchie par la surface de l'iris et rediriger vers l'extérieur un ou plusieurs faisceaux lumineux pour calculer le diamètre de la pupille.

Par « lentille sclérale », on entend ici et dans le cadre de l'invention le sens usuel, à savoir une lentille de contact de grand diamètre qui en configuration portée par l'œil passe en pont au-dessus de la cornée sans la toucher, en prenant appui sur la sclère de l'œil.

La lentille sclérale selon l'invention peut être rigide ou hybride (semi-rigide).

L'élément optique est de préférence un élément de diffraction et/ou de réfraction optique.

Selon un mode de réalisation avantageux, l'élément de diffraction optique est constitué d'un réseau de micro-optiques.

Avantageusement, le réseau de micro-optiques est constitué par des lentilles de Fresnel hors axe.

Selon un autre mode de réalisation avantageux, la lentille sclérale comprend une autre source d'illumination adaptée pour émettre un faisceau lumineux destiné à être dirigé vers l'extérieur de la membrane dans une direction à l'opposé de l'œil.

De préférence, les sources d'illumination émettent dans l'infra-rouge.

Selon une variante avantageuse de réalisation, chaque source d'illumination est un laser, de préférence à cavité verticale à émission par la surface (VCSEL), ou une diode laser à émission par la tranche.

Avantageusement, le laser VCSEL est muni d'une optique de mise en forme de son faisceau.

Selon une configuration avantageuse, la lentille sclérale comprend :
- une interface, encapsulée dans la membrane, de collecte et d'alimentation en énergie électrique de la source d'illumination et des composants actifs de la puce électronique, depuis l'extérieur de la lentille.
- au moins un circuit électronique, encapsulé dans la membrane, adapté pour activer la source d'illumination et les composants actifs de la puce électronique à partir de l'interface.

Selon un autre mode de réalisation avantageux, la lentille sclérale comprend une batterie encapsulée dans la membrane et reliée à l'interface, la batterie étant adaptée pour être rechargée depuis l'interface et pour alimenter électriquement les sources d'illumination et/ou les fonctions optoélectroniques associées aux sources d'illumination, le circuit électronique étant adapté pour activer les sources à partir de la batterie.

L'invention concerne également un pupillomètre comprenant :
- au moins une lentille sclérale selon la première alternative de l'invention ;
- un système d'acquisition de données, agencé à distance de la lentille adapté pour recevoir les informations relatives au diamètre de pupille calculé par communication sans fil et transmise par l'antenne de la lentille.

L'invention concerne encore un pupillomètre comprenant:
- au moins une lentille sclérale selon la deuxième alternative de l'invention ;
- un support, destiné à être positionné de manière fixe par rapport au visage de l'individu.
- au moins un photodétecteur, solidaire du support, pour détecter les faisceaux lumineux émis par l'élément optique de la lentille de sorte à mesurer le diamètre de la pupille en prenant en compte l'angle de déviation mesuré par le détecteur.

Selon un mode de réalisation avantageux, le pupillomètre comprend une pluralité de photodétecteurs sous la forme d'une barrette de photodiodes ou une matrice de photodétecteurs, dont les colonnes sont adaptées pour détecter les faisceaux lumineux et les lignes sont adaptées pour rendre la détection par les colonnes indépendante d'un mouvement horizontal de l'oeil.

Avantageusement, le support est une monture, destinée à être portée sur le visage de l'individu, telle qu'une monture de lunette ou un casque de réalité augmentée ou un dispositif de réalité augmentée.

Selon un autre mode de réalisation avantageux, le pupillomètre comprend au moins un détecteur, solidaire du support, le(s) détecteur(s) étant adapté(s) pour détecter la position du faisceau d'illumination de la lentille dirigé vers l'extérieur de sorte à en extraire l'angle de déviation par rapport à la normale du regard.

Ainsi, l'invention consiste essentiellement en une lentille sclérale dont la membrane intègre/encapsule au moins une source d'illumination, de préférence un laser VCSEL, qui envoie au moins un faisceau vers l'iris d'un œil. La part de faisceau lumineux réfléchi par l'iris est fonction de l'ouverture de la pupille. Ce faisceau réfléchi est récupéré par un élément encapsulé dans la membrane de la lentille et analysé par un dispositif adapté.

L'analyse peut être soit intégralement faite au sein de de la lentille sclérale qui embarque alors un photodétecteur et une puce électronique qui va calculer l'ouverture de la pupille par comparaison des données numériques issues des signaux convertis depuis le photodétecteur avec une table de correspondance préalablement déterminée, soit extérieurement à la lentille par un dispositif adéquat. Dans la première alternative, la communication du diamètre calculé est transmise vers l'extérieur de la lentille par un protocole de communication sans fil, de préférence NFC. Dans la deuxième alternative, la transmission des signaux lumineux vers l'extérieur est réalisée au moyen d'un élément optique qui va créer un ou plusieurs spots lumineux en direction de détecteurs, telles que des photodiodes agencées à distance donnée de la lentille sclérale.

Grâce à la lentille selon l'invention, la mesure de la dimension de la pupille peut s'effectuer sans la participation volontaire de l'individu qui porte la lentille sclérale, qu'il soit en mouvement ou non et sans qu'il ait besoin de fermer sa paupière comme certaines techniques de l'état de l'art.

En outre, la mesure du diamètre de pupille avec une lentille selon l'invention est rapide car elle ne nécessite pas de traitement d'images comme certaines techniques de l'état de l'art et simple.

La lentille sclérale selon l'invention intègre avantageusement des ressources d'énergie, e.g. batterie(s) ou des moyens de ressourcement, permettant aux fonctions embarquées d'opérer. Pour la première alternative, la lentille intègre de préférence des moyens de communications sans fil comme décrit dans [2], ainsi que des moyens d'activation/désactivation et transfert de données dans le sens montant de la lentille vers un système d'acquisition/ de pilotage à distance de la lentille.

Le système d'acquisition/pilotage et la détection éventuelle des spots de faisceau optique provenant de la lentille selon la deuxième alternative sont avantageusement intégrés dans une monture, tel qu'une paire de lunette, un casque de réalité mixte etc., comme décrit dans la demande de brevet FR1903979 qui permet un échange d'informations avec la lentille sclérale ainsi qu'un rechargement d'énergie à distance.

On peut intégrer d'autres fonctions à une lentille sclérale selon l'invention, telles que l'oculométrie comme décrit dans la demande de brevet FR2000575, la réfractométrie comme décrit dans la demande FR2203684, fonctions qui permettent une mesure complète des paramètres d'attitude de l'oeil.

D'autres avantages et caractéristiques de l'invention ressortiront mieux à la lecture de la description détaillée d'exemples de mise en œuvre de l'invention faite à titre illustratif et non limitatif en référence aux figures suivantes.

### Brève description des dessins

[Fig 1] la figure 1 est une vue schématique en perspective d'une lentille sclérale selon l'état de l'art, sous la forme générale d'une calotte sphérique
[Fig 2] la figure 2 est une vue schématique en coupe transversale illustrant une lentille sclérale selon une première alternative de l'invention posée sur un œil dont on cherche à mesurer le diamètre de pupille.
[Fig 3] la figure 3 une vue schématique de face d'un iris et pupille d'un œil illustrant la variation possible de diamètre de ce dernier.
[Fig 4] la figure 4 est une vue schématique en coupe transversale illustrant une lentille sclérale selon une variante de la première alternative de l'invention.
[Fig 5] la figure 5 est une vue schématique en coupe transversale illustrant une lentille sclérale selon une deuxième alternative de l'invention posée sur un œil dont on cherche à mesurer le diamètre de pupille.
[Fig 6] la figure 6 est une vue schématique illustrant un exemple d'un élément optique diffractif mis en œuvre dans l'alternative de la figure 5 utilisant un motif de type lentille de Fresnel hors axe selon quatre positions distinctes.
[Fig 7] la figure 7 est une vue schématique illustrant deux configurations de mesure de diamètre d'une pupille, obtenues à partir d'un élément optique diffractif, mis en œuvre dans l'alternative de la figure 5.
[Fig 8] la figure 8 est une vue schématique en coupe transversale illustrant une lentille sclérale selon une variante de la deuxième alternative de l'invention.
[Fig 9] la figure 9 est une vue schématique un pupillomètre avec une lentille sclérale selon l'alternative de la figure 5, un support de type lunettes intégrant au moins une barrette de photodétecteurs.
[Fig 10] la figure10 est une vue synoptique montrant le fonctionnement d'un dispositif de rechargement par induction électromagnétique permettant de recharger la batterie déformable encapsulée dans une lentille sclérale selon l'invention.
[Fig 11A], [Fig 11B] les figures 11A et 11B sont des vues montrant des étapes de réalisation successives d'une lentille sclérale selon l'invention.

### Description détaillée

Dans l'ensemble de la présente demande, les termes « interne », « externe » sont à comprendre par référence par rapport à une lentille sclérale selon en configuration portée par un œil. Ainsi, la face interne désigne la face de la lentille en contact avec la surface de l'œil tandis que la face externe désigne la face en contact avec l'extérieur.

De même, les termes « dessus », « dessous », « haut », « bas » sont à comprendre par référence par rapport à une lentille sclérale selon en configuration portée par un œil dont l'axe optique est sensiblement à l'horizontal.

Par « axe optique de l'œil », on entend ici et dans le cadre de l'invention, un axe identifié en pratique clinique comme la direction reliant un point source lumineux, et le centre des reflets lumineux des quatre surfaces réfractives de l'œil (faces antérieure et postérieure de la cornée, faces antérieure et postérieure du cristallin).

On précise que les différents éléments selon l'invention sont représentés uniquement par souci de clarté et qu'ils ne sont pas nécessairement à l'échelle.

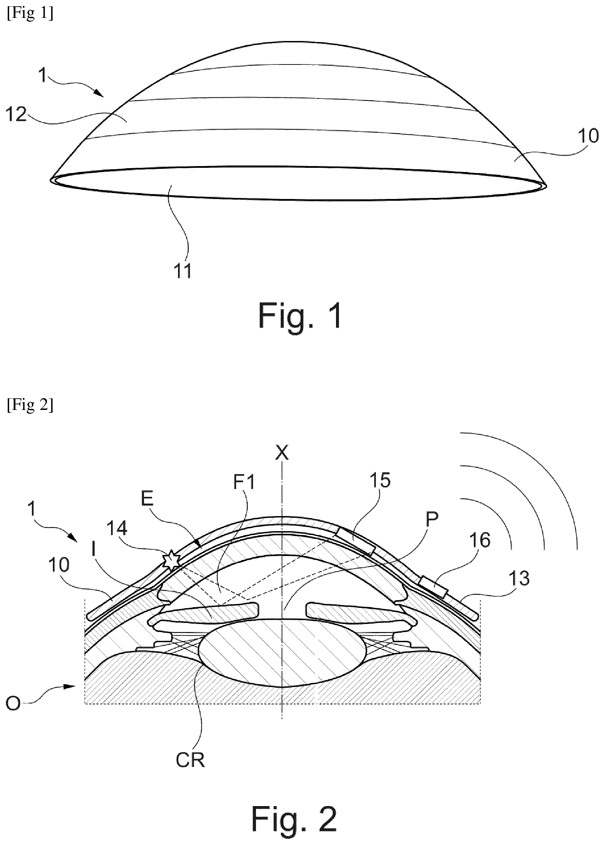

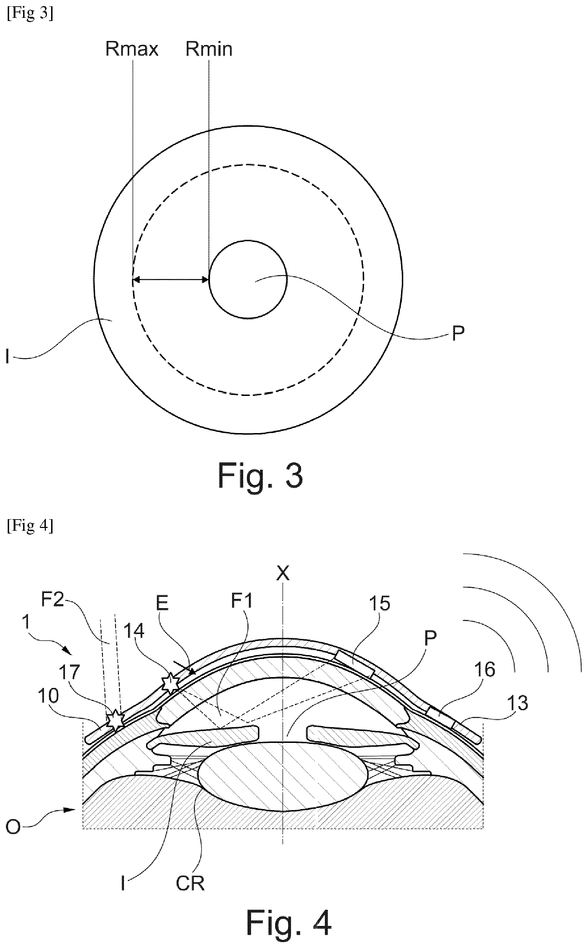

On a représenté sur les figures 2 et 4 deux alternatives distinctes d'une lentille sclérale 1 destinée à être portée par l'œil d'un individu.

Comme illustré à la figure 1, une lentille sclérale 1 présente une face interne 11 en particulier la zone d'appui avec la sclère, adaptée pour une position et une stabilisation optimales sur l'œil, et une face externe 12 d'une membrane 10 qui définissent une forme générale de calotte sphérique. Ces lentilles sclérales 1 ne sont pas en contact avec la cornée, avec un espace E typiquement de quelques centaines de microns entre la surface de la cornée et la face interne 11 de la lentille 1, et la portion périphérique 13 de la lentille 1 repose de manière régulière sur la sclère, comme montré en figures 2 et 4. Ces deux caractéristiques en font des lentilles très confortables et très stables sur l'oeil.

La lentille sclérale 1 selon la première alternative illustrée à la figure 2, est configurée pour être appliquée sur un œil O d'un individu présentant un axe optique X.

L'oeil O comporte un iris I percé en son centre par une ouverture circulaire appelée pupille P par laquelle est transmise la lumière. L'iris I se dilate ou se contracte selon l'intensité lumineuse. L'œil O comporte également un cristallin CR formé par un disque fibreux, transparent et flexible pour focaliser la lumière incidente reçue au travers de la pupille P.

Comme visible sur la figure 1, la pupille P et le cristallin CR sont sensiblement centrés sur l'axe optique X.

La lentille sclérale 1, supporte par encapsulation dans sa membrane 10, une source d'illumination 14. L'axe central de la membrane 10 est sensiblement confondu avec l'axe optique X.

La membrane transparente 10 au contact de la cornée est de préférence réalisée en matériau biocompatible par exemple à base de silicone hydrogel ou de HEMA (acronyme anglo-saxon pour « Hydroxy Ethyl Methacrylate »). Il peut s'agit de tout autre matériau bio-compatible, approprié.

La source 14 peut être un laser à cavité verticale émission par la surface (VCSEL acronyme anglo-saxon pour « Vertical-Cavity Surface-Emitting laser ») ou encore une diode laser à émission par la tranche. La lumière émise dans l'infrarouge, par cette source 14 peut être cohérente (VCSEL). De préférence, cette source 14 est un VCSEL.

Le fait que la lentille sclérale n'est pas en contact direct avec la cornée de l'œilavec l'espace E permet ainsi au faisceau F1 de la source 14 de diverger suffisamment et d'éclairer l'iris I sur une surface suffisante pour la mesure du diamètre de pupille P détaillé ci-après.

Une forme de source 14, comme par exemple une diode de forme elliptique ou la mise en place d'une optique de mise en forme sur cette source 14 peut être envisagée afin que le faisceau lumineux F1 qu'elle émet éclaire au mieux la section de l'iris I, comme décrit dans la demande de brevet FR2203684.

Une photodiode 15 est intégrée dans une puce électronique 16, également encapsulée dans la membrane 10.

Comme montré à la figure 2, la photodiode 15 est agencée de sorte à capter le faisceau F1 émis par la source d'illumination 14 et qui est réfléchie par la surface de l'iris I.

La puce électronique 16 comprend un microcontrôleur, relié au photodétecteur par le biais d'un convertisseur analogique-numérique (ADC) et ainsi adapté pour convertir en données numériques les signaux électriques issus du photodétecteur. Ainsi, le microcontrôleur peut calculer le diamètre de la pupille, à partir des données numériques converties, en fonction d'une table de correspondance prédéterminée.

La puce électronique 16 en outre est connectée à une antenne, non représentée pour transmettre les données sur le diamètre de pupille calculé par communication sans fil, vers l'extérieur de la lentille.

La communication sans fil s'effectue de préférence selon un protocole de communication en champ proche, souvent désigné par son sigle NFC (acronyme anglo-saxon pour « Near-Field Communication »).

Ainsi, selon cette première alternative, la mesure avec le calcul afférent du diamètre de la pupille P est entièrement réalisé au sein de la lentille sclérale 1, seules les informations relatives au diamètre étant transmises à l'extérieur de préférence par protocole NFC vers un support de type monture de lunettes ou un téléphone portable, porté par l'individu ou un analyseur extérieur fixe comme décrit dans [2].

On peut prévoir une interface de rechargement électrique par induction, encapsulée dans la membrane, pour la collecte et l'alimentation en énergie électrique de la source d'illumination 14 et des composants actifs de la puce électronique 16, depuis l'extérieur de la lentille, avec au moins un circuit électronique, encapsulé dans la membrane, adapté pour activer la source d'illumination et les composants actifs de la puce électronique à partir de l'interface.

Alternativement, la lentille sclérale peut intégrer au sein de sa membrane 10 une batterie autonome rechargeable qui alimente la source et les composants actifs de la puce 16 dont la photodiode 15. Cette batterie est avantageusement un accumulateur tel que décrit et revendiqué dans la demande de brevet WO2018/167393A1.

La puce électronique 16 peut par exemple être réalisée à partir d'une puce commercialisée sous la dénomination NHS3152 de la société NXP.

La figure 3 schématise l'iris I et la pupille P d'un œil O. Typiquement pour un être humain, le diamètre de la pupille P peut varier environ entre 2 et 8 mm. La source de lumière selon l'invention 14 doit donc éclairer une section de l'iris I comprise entre Rmin et Rmax. Par exemple, Rmin est égal à 1,5mm et Rmax à 3mm. La table de correspondance peut être avantageusement déterminée à partir de ces valeurs.

Afin de permettre à la source de lumière 14 d'éclairer toute la section d'iris I souhaitée, il peut être avantageux de rallonger le chemin optique parcouru par le faisceau F1 émis par la source 14. Par exemple, la source de lumière 14 peut être orientée vers l'extérieur et guidée par réflexions multiples comme décrit dans la demande de brevet FR2000575.

La figure 4 illustre une variante de la première alternative, selon laquelle on réalise en plus un suivi de pointage précis de la direction du regard.

Pour ce faire, une source d'illumination supplémentaire 17 est encapsulée dans la membrane 10.

Le faisceau F2 émis par cette autre source 17 éclaire vers l'extérieur afin de réaliser un pointage optique qui permet de connaître la direction du regard. On peut avantageusement mettre en œuvre la réalisation du faisceau F2 et sa détection comme décrit dans la demande de brevet WO2020/212394.

Le faisceau lumineux F2 peut être analysé par un détecteur sensible à la position (PSD) qui peut être en fonction des configurations embarquée dans la lentille et/ou dans un support fixe par rapport à l'œil de l'individu. La connaissance de la direction du regard permet de compenser l'alignement variable de l'œil avec le PSD.

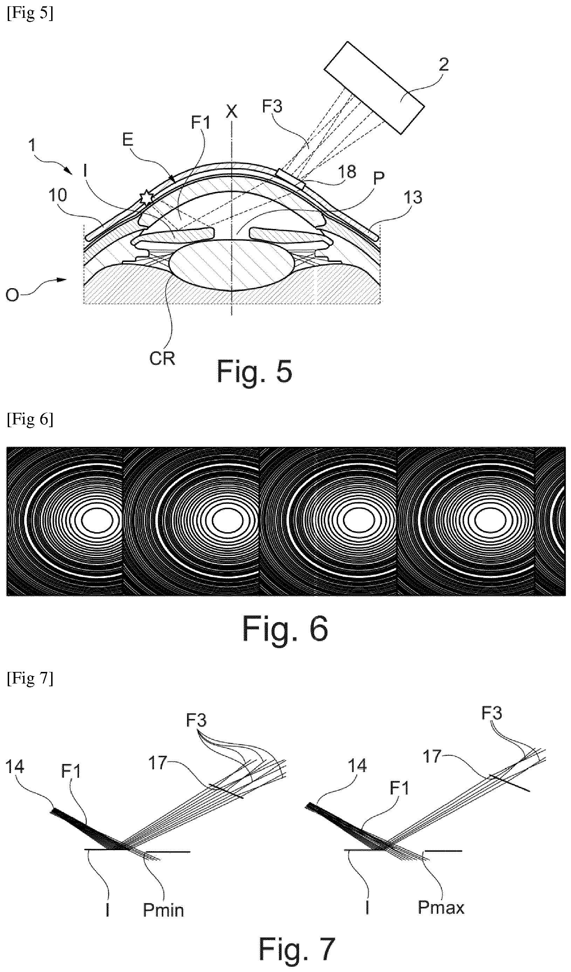

La lentille sclérale 1 selon la figure 5 met en œuvre une deuxième alternative de l'invention selon laquelle le faisceau lumineux F1 émis par la source d'illumination 14 et réfléchi par l'iris I n'est plus analysé au sein même de la lentille comme selon la figure 2 mais extérieurement à celle-ci par un dispositif de détection 2 détaillé par la suite.

Ainsi, la lentille 1 comprend ici un élément optique, de préférence un élément optique diffractif 18 est encapsulé dans la membrane 10 dans une zone en regard de l'iris I, de préférence en regard de la zone de mydriase).

En fonction de la proportion du faisceau de lumière F1 émis par la source 14 qui sera réfléchie par la portion d'iris I, l'élément optique 18 sera plus ou moins éclairé.

L'élément optique diffractif 18 est constitué de plusieurs facettes, chacune donnant lieu à la création d'un faisceau lumineux F3 avec une puissance de focalisation, qui se formera à une distance donnée de la lentille 1 où est agencé un ou plusieurs détecteurs 2, de préférence un ou des détecteurs à quadrant.

Ces détecteurs 2 peuvent par exemple des photodiodes quadrants comme celles commercialisées par la société First sensor :
www.first-sensor.com/fr/produits/capteurs-optiques/detecteurs/apd-quadrants-qa/.

Le détecteurs 2 sont de préférence sous la forme d'une ou plusieurs barrettes de photodiode.

La détection de chacun des faisceaux lumineux F3 par le ou les détecteurs 2 extérieurs à la lentille 1 permet de déterminer le diamètre d'ouverture de l'iris.

L'élément optique diffractif 18 peut avantageusement être constitué par un réseau de micro-optiques, tel qu'un réseau de prismes ou un réseau échelette, aussi appelé réseau « blazé », ou d'une pluralité de lentilles de Fresnel hors axe, i.e. qui intègrent chacune une fonction de déviation de sorte que chaque secteur de l'élément optique 18 s'image sur une photodiode donnée de la barrette de photodiodes, différente de celles sur lesquelles s'imagent les autres secteurs de l'élément optique 18. La réflexion sur la surface semi-diffusante de l'iris ayant pour effet de réduire la cohérence de la source d'illumination 14, ce type de composant est bien adapté pour la création des spots lumineux F3 recherchée.

La figure 6 illustre un exemple d'un élément optique diffractif 18 mis en œuvre dans l'alternative de la figure 5 utilisant un motif de type lentille de Fresnel hors axe, c'est-à-dire avec une focalisation avec un décalage axial, selon quatre positions distinctes.

La figure 7 illustre deux configurations d'interception de la surface d'iris I par le faisceau F1 émis par la source d'illumination 14 en fonction de l'ouverture de la pupille (pupille ouverte au minimum Pmin sur la gauche, au maximum Pmax sur la droite). Les microlentilles mises en œuvre en tant qu'élément optique diffractif 18, forment donc ici 1 à 4 points de focalisation en fonction de la taille de la pupille et donc la surface d'iris I éclairée par le faisceau F1. On précise que sur cette figure 7, par simplicité, la cornée n'est pas représentée : en effet, l'indice de réfraction égal à 1,336 pour l'humeur aqueuse, 1,376 pour la cornée et 1,33 pour le fluide lacrymal étant proches, l'influence de la puissance réfractive de la cornée sur le tracé est négligeable, si l'angle d'incidence ne s'éloigne pas trop de la normale.

La deuxième alternative de l'invention peut également mettre en œuvre un suivi de pointage précis de la direction du regard.

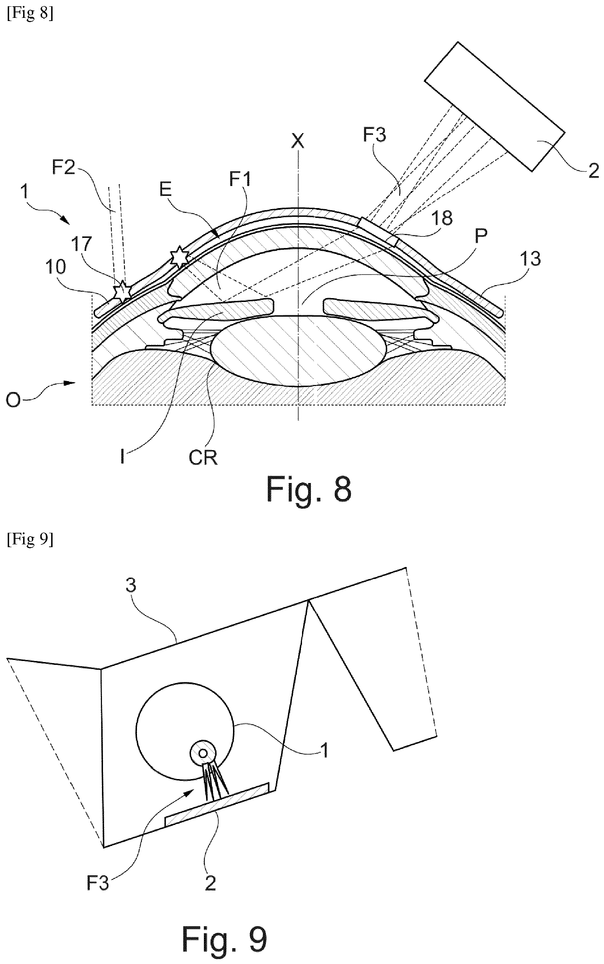

Aussi, comme illustré en figure 8, une source d'illumination supplémentaire 17 est encapsulées dans la membrane 10.

Le faisceau F2 émis par cette autre source 17 éclaire vers l'extérieur afin de réaliser un pointage optique qui permet de connaître la direction du regard. On peut avantageusement mettre en œuvre la réalisation du faisceau F2 et sa détection comme décrit dans la demande de brevet WO2020/212394.

Le faisceau lumineux F2 peut être analysé, par exemple, par un détecteur sensible à la position (PSD) qui peut être en fonction des configurations embarquée dans la lentille et/ou dans un support fixe par rapport à l'œil de l'individu. La connaissance de la direction du regard permet de compenser l'alignement variable de l'œil avec le PSD.

De manière générale, aucune source d'illumination 14, 17 embarquée par la lentille 1 ni aucun des composants électroniques 15, 16 ou élément optique 18 embarqués par la lentille 1 ne bloque la vue de l'individu.

Le ou les détecteurs 2 pour la détection des faisceaux F3 sont avantageusement placés autour de l'œil, de préférence sur un support embarqué par l'individu tel que des lunettes 3, comme représenté de manière schématique à la figure 9. Typiquement, il peut s'agir d'une barrette de détecteurs 2, par exemple des photodiodes agencées à quelques centimètres de la lentille sclérale 1 en configuration de mesure. La détection des faisceaux F3 sur chacun de ces détecteurs 2 permet ainsi de remonter à l'ouverture de la pupille P.

On peut aussi remplacer la barrette 2 par une matrice de détecteurs 2 couvrant par exemple un secteur angulaire donné, typiquement de l'ordre de + ou - 15° autour de la lentille sclérale 1. Une telle matrice permet également d'intégrer un mouvement horizontal de l'œil O.

Dans le cas où la lentille sclérale selon l'invention embarque une batterie souple pour l'alimentation de l'ensemble des composants électroniques/optoélectroniques, on prévoit avantageusement un système de rechargement par induction magnétique de cette batterie.

Ainsi, de préférence une antenne sous la forme d'une bobine d'induction 19, reliée un redresseur sont encapsulés dans la membrane 10 d'une lentille sclérale 1.

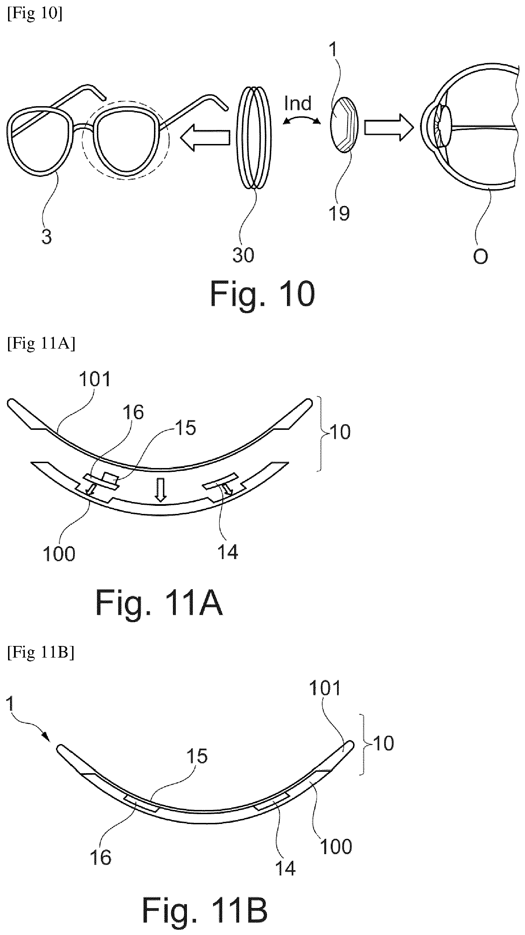

Un exemple avantageux de système de rechargement est montré en figure 10: une antenne d'induction 30 est intégrée dans une monture de lunettes 3, de préférence qui supporte les détecteurs 2 et le cas échéant un détecteur sensible à la position PSD pour détecter le faisceau F2. L'antenne 30 transfère de l'énergie par couplage magnétique à l'antenne 19 de la lentille de contact 1 qui peut être en place sur l'œilO d'un individu pendant la recharge par induction magnétique. On pourra se reporter à la publication [2] pour plus de détails.

Les figures 11A et 11B illustrent certaines étapes d'un procédé de réalisation d'une lentille sclérale selon la première alternative de l'invention.

La membrane 10 est ici constituée de deux films 100, 101 en polymère transparent, par exemple un hydrogel.

Chacun des deux films 100, 101 est tout d'abord mis en forme comme usuellement.

Puis, toute l'électronique, à l'exception éventuellement de l'antenne de collecte d'énergie par induction, est mise en place sur la face intérieure du film extérieur 100.

Ainsi, l'électronique dont la source d'illumination 14 et la puce électronique 16 avec la photodiode 15, est parfaitement positionnée au sein du film 100.

Une fois, ce positionnement effectué, les deux films 100, 101 en polymère transparent sont scellés entre eux, par colle UV par exemple.

Ainsi, tous les composants électroniques ou optoélectroniques sont parfaitement positionnés et encapsulés entre les deux films 100, 101.

Le même procédé peut être mis en œuvre pour encapsuler la source d'illumination 14 et l'élément diffractif 18 ainsi que l'électronique.

D'autres variantes et améliorations peuvent être apportées sans pour autant sortir du cadre de l'invention.

Si dans les exemples illustrés, le photodétecteur 15 ou l'élément optique diffractif 18 sont agencés au sein de la membrane 10 de l'autre côté de la pupille P par rapport à la source de lumière 14, on peut tout aussi bien envisager de les agencer côte-à-côte.

De même, si dans les exemples des figures 4 et 8, la source 17 permettant de connaître la direction du regard est agencée côte-à-côte avec la source d'illumination 14 divergeant vers l'iris, on peut aussi envisager de la placer de l'autre côté de la pupille P, par exemple à côté de l'élément optique diffractif 18.

En lieu et place ou en sus d'un élément diffractif 18, on peut envisager d'avoir un élément optique réfractif.

### Liste des références citées

[1] M. Larson, M. Behrends, « Portable Infrared Pupillometry: A Review », Anesthesia and Analgesia 120(6):1242-53 DOI:10.1213/ANE.0000000000000314, (2015).
[2] A. Khaldi, E. Daniel, L. Massin, C. Kärnfelt, F. Ferranti, C. Lahuec, F. Seguin, V. Nourrit, J-L de Bougrenet de la Tocnaye, "The cyclops contact lens: A laser emitting contact lens for eye tracking", Scientific Report 10, 14804, doi.org/10.1038/s41598-020-71233-1, (2020).
[3] Applied Digital Optics: "From Micro-optics to Nanophotonics (Chapter 6), "Bernard C. Kress, Patrick Meyrueis, Wiley, November 2009, ISBN: 978-0-470-02264-1.

## Revendications

1. Lentille sclérale (1) pour la mesure du diamètre d'une pupille d'œil d'individu, comprenant:
- une membrane (10) adaptée pour recouvrir la pupille, l'iris et au moins partiellement la sclère de l'œil;
- une source d'illumination (14) encapsulée dans la membrane, la source d'illumination étant adaptée pour émettre un cône ou faisceau lumineux (F1) destiné à diverger directement ou indirectement vers l'iris (I) ;
- un circuit électronique (16), encapsulée dans la membrane, comprenant au moins comme composants:
au moins un photodétecteur (15) agencé de sorte à capter le faisceau émis par la source d'illumination réfléchie par la surface de l'iris,
un microcontrôleur, relié au photodétecteur et adapté pour convertir en données numériques les signaux électriques issus du photodétecteur et calculer le diamètre de la pupille, à partir des données numériques converties, en fonction d'une table de correspondance prédéterminée, et les coder pour transmission par communication sans fil;
une antenne pour transmettre les informations relatives au diamètre de pupille calculé par communication sans fil.

2. Lentille sclérale (1) selon la revendication 1, le photodétecteur étant une photodiode.

3. Lentille sclérale (1) selon la revendication 1 ou 2, l'antenne étant adaptée pour transmettre les informations par communication en champs proche (NFC).

4. Lentille sclérale (1) selon l'une des revendications précédentes, l'antenne étant en outre adaptée pour réaliser une recharge électrique de la source d'illumination et/ou des composants actifs du circuit électronique.

5. Lentille sclérale (1) pour la mesure du diamètre d'une pupille d'œil d'individu, comprenant:
- une membrane (10) adaptée pour recouvrir la pupille, l'iris et au moins partiellement la sclère de l'œil;
- une source d'illumination (14), encapsulée dans la membrane et adaptée pour émettre un cône ou faisceau lumineux (F1) destiné à diverger directement ou indirectement vers l'iris (I) ;
- un élément optique (18) encapsulé dans la membrane et agencé de sorte à capter le faisceau émis par la source d'illumination réfléchie par la surface de l'iris et rediriger vers l'extérieur un ou plusieurs faisceaux lumineux (F3) pour calculer le diamètre de la pupille (P).

6. Lentille sclérale (1) selon la revendication 5, l'élément optique étant un élément de diffraction et/ou de réfraction optique.

7. Lentille sclérale (1) selon la revendication 6, l'élément de diffraction optique étant constitué d'un réseau de micro-optiques.

8. Lentille sclérale (1) selon la revendication 7, le réseau de micro-optiques étant constitué par des lentilles de Fresnel hors axe.

9. Lentille sclérale (1) selon l'une des revendications précédentes, comprenant une autre source d'illumination (17) adaptée pour émettre un faisceau lumineux (F2) destiné à être dirigé vers l'extérieur de la membrane dans une direction à l'opposé de l'œil.

10. Lentille sclérale (1) selon l'une des revendications précédentes, les sources d'illumination (14, 17) émettant dans l'infra-rouge.

11. Lentille sclérale (1) selon l'une des revendications précédentes, chaque source d'illumination étant un laser, de préférence à cavité verticale à émission par la surface (VCSEL), ou une diode laser à émission par la tranche.

12. Lentille sclérale (1) selon la revendication 11, le laser VCSEL étant muni d'une optique de mise en forme de son faisceau.

13. Lentille sclérale (1) selon l'une des revendications précédentes, comprenant :
- une interface, encapsulée dans la membrane, de collecte et d'alimentation en énergie électrique de la source d'illumination et des composants actifs de la puce électronique, depuis l'extérieur de la lentille.
- au moins un circuit électronique, encapsulé dans la membrane, adapté pour activer la source d 'illumination et les composants actifs de la puce électronique à partir de l'interface.

14. Lentille sclérale (1) selon la revendication 13, comprenant une batterie encapsulée dans la membrane et reliée à l'interface, la batterie étant adaptée pour être rechargée depuis l'interface et pour alimenter électriquement les sources d'illumination et/ou les fonctions optoélectroniques associées aux sources d'illumination, le circuit électronique étant adapté pour activer les sources à partir de la batterie.

15. Pupillomètre comprenant :
- au moins une lentille sclérale selon l'une des revendications précédentes à l'exception des revendications 5 à 8;
- un système d'acquisition de données, agencé à distance de la lentille adapté pour recevoir les informations relatives au diamètre de pupille calculé par communication sans fil et transmise par l'antenne de la lentille.

16. Pupillomètre comprenant :
- au moins une lentille sclérale selon l'une des revendications 9 à 14;
- un support (3), destiné à être positionné de manière fixe par rapport au visage de l'individu.
- au moins un photodétecteur (2), solidaire du support, pour détecter les faisceaux lumineux (F3) émis par l'élément optique (18) de la lentille de sorte à mesurer le diamètre de la pupille en prenant en compte l'angle de déviation mesuré par le détecteur.

17. Pupillomètre selon la revendication 16, comprenant une pluralité de photodétecteurs sous la forme d'une barrette de photodiodes ou une matrice de photodétecteurs, dont les colonnes sont adaptées pour détecter les faisceaux lumineux (F3) et les lignes sont adaptées pour rendre la détection par les colonnes indépendante d'un mouvement horizontal de l'œil.

18. Pupillomètre selon l'une des revendications 16 ou 17, le support étant une monture (3), destinée à être portée sur le visage de l'individu, telle qu'une monture de lunette ou un casque de réalité augmentée ou un dispositif de réalité augmentée.

19. Pupillomètre selon l'une des revendications 15 à 18, comprenant au moins un détecteur, solidaire du support, le(s) détecteur(s) étant adapté(s) pour détecter la position du faisceau d'illumination de la lentille (F2) dirigé vers l'extérieur de sorte à en extraire l'angle de déviation par rapport à la normale du regard.

## Patentansprüche

1. Sklerallinse (1) zur Messung des Durchmessers einer Pupille eines Auges einer Person, beinhaltend:
- eine Membran (10), die dazu angepasst ist, die Pupille, die Iris und mindestens teilweise die Sklera des Auges zu bedecken;
- eine Beleuchtungsquelle (14), die in der Membran verkapselt ist, wobei die Beleuchtungsquelle dazu angepasst ist, einen Lichtkegel oder -strahl (F1) zu emittieren, der dazu bestimmt ist, direkt oder indirekt zu der Iris (I) zu divergieren;
- eine elektronische Schaltung (16), die in der Membran verkapselt ist und als Komponenten mindestens Folgendes beinhaltet:
mindestens einen Fotodetektor (15), der so eingerichtet ist, dass er den durch die Beleuchtungsquelle emittierten und durch die Oberfläche der Iris reflektierten Strahl erfasst,
einen Mikrocontroller, der mit dem Fotodetektor verbunden ist und dazu angepasst ist, die aus dem Fotodetektor stammenden elektrischen Signale in digitale Daten umzuwandeln und anhand der umgewandelten digitalen Daten den Durchmesser der Pupille in Abhängigkeit von einer vorbestimmten Nachschlagetabelle zu berechnen und sie für eine Übertragung per drahtloser Kommunikation zu codieren;
eine Antenne, um die Informationen über den berechneten Pupillendurchmesser per drahtloser Kommunikation zu übertragen.

2. Sklerallinse (1) nach Anspruch 1, wobei der Fotodetektor eine Fotodiode ist.

3. Sklerallinse (1) nach Anspruch 1 oder 2, wobei die Antenne dazu angepasst ist, die Informationen per Nahfeldkommunikation (NFC) zu übertragen.

4. Sklerallinse (1) nach einem der vorhergehenden Ansprüche, wobei die Antenne ferner dazu angepasst ist, eine elektrische Wiederaufladung der Beleuchtungsquelle und/oder der aktiven Komponenten der elektronischen Schaltung durchzuführen.

5. Sklerallinse (1) zur Messung des Durchmessers einer Pupille eines Auges einer Person, beinhaltend:
- eine Membran (10), die dazu angepasst ist, die Pupille, die Iris und mindestens teilweise die Sklera des Auges zu bedecken;
- eine Beleuchtungsquelle (14), die in der Membran verkapselt ist und dazu angepasst ist, einen Lichtkegel oder -strahl (F1) zu emittieren, der dazu bestimmt ist, direkt oder indirekt zu der Iris (I) zu divergieren;
- ein optisches Element (18), das in der Membran verkapselt ist und so eingerichtet ist, dass es den durch die Beleuchtungsquelle emittierten und durch die Oberfläche der Iris reflektierten Strahl erfasst und einen oder mehrere Lichtstrahlen (F3) nach außen weiterleitet, um den Durchmesser der Pupille (P) zu berechnen.

6. Sklerallinse (1) nach Anspruch 5, wobei das optische Element ein diffraktives und/oder refraktives optisches Element ist.

7. Sklerallinse (1) nach Anspruch 6, wobei das diffraktive optische Element aus einem Gitter aus Mikrooptiken besteht.

8. Sklerallinse (1) nach Anspruch 7, wobei das Gitter aus Mikrooptiken aus außeraxialen Fresnel-Linsen besteht.

9. Sklerallinse (1) nach einem der vorhergehenden Ansprüche, beinhaltend eine weitere Beleuchtungsquelle (17), die dazu angepasst ist, einen Lichtstrahl (F2) zu emittieren, der dazu bestimmt ist, in einer zum Auge entgegengesetzten Richtung zur Außenseite der Membran hin gerichtet zu sein.

10. Sklerallinse (1) nach einem der vorhergehenden Ansprüche, wobei die Beleuchtungsquellen (14, 17) im Infrarotbereich emittieren.

11. Sklerallinse (1) nach einem der vorhergehenden Ansprüche, wobei jede Beleuchtungsquelle ein Laser, vorzugsweise ein oberflächenemittierender Vertikalresonatorlaser (VCSEL), oder eine kantenemittierende Laserdiode ist.

12. Sklerallinse (1) nach Anspruch 11, wobei der VCSEL-Laser über eine Formungsoptik für seinen Strahl verfügt.

13. Sklerallinse (1) nach einem der vorhergehenden Ansprüche, beinhaltend:
- eine Schnittstelle, die in der Membran verkapselt ist, zur Sammlung von und zur Versorgung der Beleuchtungsquelle und der aktiven Komponenten des elektronischen Chips mit elektrischer Energie von der Außenseite der Linse her.
- mindestens eine elektronische Schaltung, die in der Membran verkapselt ist und dazu angepasst ist, die Beleuchtungsquelle und die aktiven Komponenten des elektronischen Chips mittels der Schnittstelle zu aktivieren.

14. Sklerallinse (1) nach Anspruch 13, beinhaltend eine Batterie, die in der Membran verkapselt ist und mit der Schnittstelle verbunden ist, wobei die Batterie dazu angepasst ist, von der Schnittstelle aus wiederaufgeladen zu werden und die Beleuchtungsquellen und/oder die mit den Beleuchtungsquellen assoziierten optoelektronischen Funktionen mit Strom zu versorgen, wobei die elektronische Schaltung dazu angepasst ist, die Quellen mittels der Batterie zu aktivieren.

15. Pupillometer, beinhaltend:
- mindestens eine Sklerallinse nach einem der vorhergehenden Ansprüche mit Ausnahme der Ansprüche 5 bis 8;
- ein Datenerfassungssystem, das von der Linse entfernt eingerichtet ist und dazu angepasst ist, die Informationen über den berechneten Pupillendurchmesser per drahtloser Kommunikation, die durch die Antenne der Linse übertragen wird, zu empfangen.

16. Pupillometer, beinhaltend:
- mindestens eine Sklerallinse nach einem der Ansprüche 9 bis 14;
- einen Träger (3), der dazu bestimmt ist, in Bezug auf das Gesicht der Person fest positioniert zu werden.
- mindestens einen Fotodetektor (2), der mit dem Träger fest verbunden ist, zur Detektion der durch das optische Element (18) der Linse emittierten Lichtstrahlen (F3), um den Durchmesser der Pupille unter Berücksichtigung des durch den Detektor gemessenen Ablenkwinkels zu messen.

17. Pupillometer nach Anspruch 16, beinhaltend eine Vielzahl von Fotodetektoren in Form eines Fotodioden-Arrays oder einer Fotodetektormatrix, dessen/deren Spalten dazu angepasst sind, die Lichtstrahlen (F3) zu detektieren, und dessen/deren Zeilen dazu angepasst sind, die Detektion durch die Spalten von einer horizontalen Bewegung des Auges unabhängig zu machen.

18. Pupillometer nach einem der Ansprüche 16 oder 17, wobei der Träger ein Gestell (3) ist, das dazu bestimmt ist, auf dem Gesicht der Person getragen zu werden, wie etwa ein Brillengestell oder ein Augmented-Reality-Helm und eine Augmented-Reality-Vorrichtung.

19. Pupillometer nach einem der Ansprüche 15 bis 18, beinhaltend mindestens einen Detektor, der mit dem Träger fest verbunden ist, wobei der oder die Detektoren dazu angepasst sind, die Position des Beleuchtungsstrahls der Linse (F2), der zur Außenseite gerichtet ist, zu detektieren, um daraus den Ablenkwinkel in Bezug auf die Normale des Blicks zu extrahieren.

## Claims

1. Scleral lens (1) for measuring the diameter of a pupil of an individual's eye, comprising:
- a membrane (10) configured to cover the pupil, the iris and at least partially the sclera of the eye;
- a light source (14) encapsulated in the membrane, the light source being configured to emit a light cone or beam (F1) intended to diverge directly or indirectly towards the iris (I);
- an electronic circuit (16), encapsulated in the membrane and comprising at least as components:
at least one photodetector (15) arranged to capture the beam emitted by the light source reflected from the surface of the iris;
a microcontroller, connected to the photodetector and configured to convert into digital data electrical signals generated by the photodetector and to calculate, based on the converted digital data, the diameter of the pupil using a predetermined look-up table, and to encode it for transmission by wireless communication;
an antenna for transmitting information relating to the calculated pupil diameter by wireless communication.

2. Scleral lens (1) according to Claim 1, wherein the photodetector is a photodiode.

3. Scleral lens (1) according to Claim 1 or 2, wherein the antenna is configured to transmit the information via near-field communication (NFC).

4. Scleral lens (1) according to any of the preceding claims, wherein the antenna is further configured to electrically recharge the light source and/or active components of the electronic circuit.

5. Scleral lens (1) for measuring the diameter of a pupil of an individual's eye, comprising:
- a membrane (10) configured to cover the pupil, the iris and at least partially the sclera of the eye;
- a light source (14), encapsulated in the membrane and configured to emit a light cone or beam (F1) intended to diverge directly or indirectly towards the iris (I);
- an optical element (18) encapsulated in the membrane and arranged to capture the beam emitted by the light source reflected by the surface of the iris and to redirect towards the exterior one or more light beams (F3) for calculating the diameter of the pupil (P).

6. Scleral lens (1) according to Claim 5, wherein the optical element is a diffractive and/or refractive optical element.

7. Scleral lens (1) according to Claim 6, wherein the diffractive optical element consists of a microlens array.

8. Scleral lens (1) according to Claim 7, wherein the microlens array is made up of off-axis Fresnel lenses.

9. Scleral lens (1) according to any of the preceding claims, comprising another light source (17) configured to emit a light beam (F2) intended to be directed towards the exterior of the membrane in a direction away from the eye.

10. Scleral lens (1) according to any of the preceding claims, wherein the light sources (14, 17) emit in the infrared.

11. Scleral lens (1) according to any of the preceding claims, wherein each light source is a laser, preferably a vertical-cavity surface-emitting laser (VCSEL), or an edge-emitting laser diode.

12. Scleral lens (1) according to Claim 11, wherein the VCSEL is equipped with an optical system for shaping its beam.

13. Scleral lens (1) according to any of the preceding claims, comprising:
- an interface, encapsulated in the membrane, for collecting and supplying electrical energy to the light source and the active components of the electronic chip, from outside the lens;
- at least one electronic circuit, encapsulated in the membrane and configured to activate the light source and the active components of the electronic chip via the interface.

14. Scleral lens (1) according to Claim 13, comprising a battery encapsulated in the membrane and connected to the interface, wherein the battery is configured to be recharged via the interface and to electrically power the light sources and/or optoelectronic functions associated with the light sources, the electronic circuit being configured to activate the sources via the battery.

15. Pupillometer, comprising:
- at least one scleral lens according to any of the preceding claims with the exception of Claims 5 to 8;
- a data acquisition system, arranged remotely from the lens and configured to receive the information relating to the calculated pupil diameter transmitted by the lens antenna by wireless communication.

16. Pupillometer, comprising:
- at least one scleral lens according to any of Claims 9 to 14;
- a carrier (3), intended to be fixedly positioned with respect to the face of the individual;
- securely fastened to the carrier, at least one photodetector (2) for detecting the light beams (F3) emitted by the optical element (18) of the lens so as to measure the diameter of the pupil based on the angle of deviation measured by the detector.

17. Pupillometer according to Claim 16, comprising a plurality of photodetectors taking the form of a strip of photodiodes or a photodetector array, the columns of which are configured to detect the light beams (F3) and the rows of which are configured to make the detection by the columns independent of a horizontal movement of the eye.

18. Pupillometer according to either of Claims 16 and 17, wherein the carrier is a mounting (3), intended to be worn on the face of the individual, such as a spectacle frame or an augmented-reality headset or an augmented-reality device.

19. Pupillometer according to any of Claims 15 to 18, comprising at least one detector, securely fastened to the carrier, wherein the at least one detector is configured to detect the position of the light beam of the lens (F2) directed towards the exterior so as to extract therefrom the angle of deviation with respect to normal of the gaze.
